# EUROPEAN PATENT APPLICATION

(11) **EP 4 059 612 A2**
(43) Date of publication of application: **21.09.2022**
(21) Application number: 20907807.0
(22) Date of filing: 22.12.2020
(51) Int. Cl.: B04B 7/00, B04B 11/00

(54) **TARGET MATERIAL EXTRACTION KIT FOR CENTRIFUGAL SEPARATOR, AND METHOD FOR EXTRACTING TARGET MATERIAL BY USING SAME**

(30) Priority: 23.12.2019 KR 20190172784
(71) Applicant: Glofinn Co., Ltd., Cheonan-si, Chungcheongnam-do 31253 (KR)
(72) Inventor: KIM, Hong, Seoul 06588 (KR)
(74) Representative: Loo, Chi Ching
(86) International application number: PCT/KR2020/018872
(87) International publication number: WO 2021/133024

(57) **Abstract**

The present invention relates to a target material extraction kit for a centrifugal separator and a method for extracting a target material by using the target material extraction kit for a centrifugal separator, and more specifically, to a target material extraction kit for a centrifugal separator and a method for extracting a target material by using the target material extraction kit for a centrifugal separator by which the target material can be more easily extracted from a source material that is separated into layers by the centrifugal separator, and thus simplification of the extraction operation of the target material results in improvement in easiness and efficiency of the extraction operation.

## Description

### Technical Field

The present invention relates to a target material extraction kit for a centrifugal separator and a method for extracting a target material by using the target material extraction kit for a centrifugal separator, and more specifically, to a target material extraction kit for a centrifugal separator and a method for extracting a target material by using the target material extraction kit for a centrifugal separator by which the target material can be more easily extracted from a source material that is separated into layers by the centrifugal separator, and thus simplification of the extraction operation of the target material results in improvement in easiness and efficiency of the extraction operation.

### Background Art

The present invention is an improved invention of'a centrifugal separation kit and a target material extraction method using the same', which was filed on May 31, 2013 and registered on September 24, 2015 by the present applicant.

The present invention provides an extraction kit for extracting a specific target material from a source material by centrifuging a source material such as whole blood or bone marrow etc., and a target material extraction method using the same.

It is an invention that maximizes the ease and efficiency of the extraction of the target material by solving the disadvantages and problems that occurred in the conventional extraction kit (briefly, the present invention simplifies the extraction of the target material.

At this time, in contrast to the present invention, the disadvantages and problems of the conventional extraction kit are as follows.
1. After centrifugation, since only the red blood cells are collected in the centrifuge tube,
2. all of the red blood cells remaining inside the syringe should be dropped to the bottom,
3. After loading all red blood cells, the syringe should be centrifuged again,
4. In order to extract the final target material, it is necessary to move the separated material other than the final target material with a separate syringe using a needle, and
5. a separate tool (a cap sealing the syringe for centrifugation again, a syringe coupler for connecting the syringe and other syringe to move the final target material to a separate syringe, etc.) is required for the extraction operation as described above.

That is, in brief, two centrifugations are required to obtain the final target material.

A dropping operation of dropping red blood cells and a needling operation of collecting the materials separated by the centrifugation using a needle in addition to the target material are necessary.

Therefore, in contrast to the present invention, in the target material extraction operation, relatively long working hours are required in the conventional art.

Also, due to the dropping, the work thereof is not clean in terms of aesthetics and environment.

In the conventional art, the overall extraction operation is cluttered, the operation sequence is complicated, and there is a possibility that the target material could be contaminated due to multiple airtightness, centrifugation, and dropping.

Therefore, in order to solve the problems with the conventional extraction kit as described above, the improved target material extraction kit is needed to simplify the extraction operation sequence for extracting the target material and to obtain the target material more reliably and easily and the present invention intends to provide it.

Accordingly, as the prior art related to a centrifuge-type target material extraction kit and a target material extraction method using the same, first, "a centrifugal separation kit and a target material extraction method using the same" of Korean Patent registration No. 10-1557190 (hereinafter referred to as "Patent Literature 1") is disclosed as shown in FIG. 6(a).

Patent Literature 1 relates to a centrifugal kit used when whole blood or marrow is centrifuged and a method for extracting a target material using the same. The centrifugal kit comprises a syringe accommodating a source material and collecting a target material discriminated through centrifuging and a centrifugal tube having an accommodating space formed therein to accommodate materials other than the target material discriminated through centrifuging and mounted on a mounting unit of the syringe. The syringe includes a hanging projection formed on the mounting unit to have a bottom outer diameter smaller than a top outer diameter. The centrifugal tube comprises a coupling hole, wherein a portion having a small outer diameter based on the hanging projection from the mounting unit is inserted and adhered to be connected with the accommodating space; and a coupling groove extended from a combining hole to have a larger inner diameter than the coupling hole and formed to accommodate a portion having a large outer diameter based on the hanging projection from the mount unit, wherein the hanging projection is hung on the bottom surface thereof. In a centrifuging and collecting step of extracting platelets or stem cells from a source material such as blood or marrow, a step for collection and extraction is simplified, so that work time can be reduced. Also, an extracted target material can be immediately used in a procedure, so that procedure time can be reduced.

As further prior art, "a kit of centrifuge separation and methods for centrifuging using the same" of Korean Patent registration No. 10-1069877 (hereinafter referred to as "Patent Literature 2") is disclosed as shown in FIG. 6(b).

Patent Literature 2 relates to a kit of centrifuge separation and methods for centrifuging using the same capable of easily extracting the target material by installing a syringe in the centrifuge tube and directly collecting the target material (Platelet-Rich Plasma or stem cells) separated through the centrifugation with the syringe, after collection of whole blood or body fluids.

As described above, Patent Literatures 1 and 2 are the same technical field as the present invention and there are substantially similar and identical technical concepts in the subject matters and solution to be solved by the invention with the present invention. However, theses, of course, are necessary configurations in order to form the extraction kit in the same technical field.

In addition, Patent Literatures 1 and 2 are technologies filed by the applicant in 2013 and 2009, respectively and technical characteristics thereof may be substantially similar or identical with the present invention.

However, the present invention, in addition to the extraction kit based on Patent Literatures 1 and 2, further simplifies the method of extracting the target material using the extraction kit and is intended to enable the extraction of the target material more perfectly.

Therefore, the present invention is different from the related art of the prior art including Patent Literatures 1 and 2, and seeks its technical characteristics, based on the subject matters to be solved by the invention (object of the invention), means (components) for solving it, and the better effect exerted by it.

### Patent Literature

Patent Literature 1: Korean Patent Registration No. 10-1557190 (September 24, 2015)
Patent Literature 2: Korean Patent Registration No. 10-1069877 (September 27, 2011)

### Disclosure

### Technical Problem

In this respect, the present invention is made to solve the above-described problem in the related art, and an object thereof is to provide an extraction kit that can easily extract a target material from a source material.

Another object of the present invention is to provide a method for easily extracting a target material from a source material by using an extraction kit.

### Technical solution

According to one aspect of the present invention so as to accomplish these objects, there is provided to a target material extraction kit for a centrifugal separator, including: a source material collecting means that collects a certain amount of source material; and a target material extracting means that is coupled and fixed to an end portion of the source material collecting means and is inserted into the centrifugal separator together with the source material collecting means, wherein the target material is to be easily separated and extracted from the source material separated into the layers formed inside the source material collecting means and the target material extracting means by the centrifugal separator.

At this time, the source material collecting means is configured to include: a source material collecting barrel unit which is to be filled with the certain amount of source material; and a source material collecting plunger unit which is inserted into the source material collecting barrel unit, moves forward and backward, and generates pressure by moving forward and backward to cause the source material collecting barrel unit to be filled with the certain amount of source material, wherein the source material collecting barrel unit is configured to include: a source material collecting space module in which the certain amount of source material is stored; and a target material extracting means coupling module which is formed to project from an end portion of the source material collecting space module such that a part of the target material extracting means coupling module is inserted into and coupled to the target material extracting means, wherein the source material collecting plunger unit is configured to include: a first airtight packing element which seals an inside of the source material collecting barrel unit to form the airtight inside; a plunger element which moves the first airtight packing element forward and backward inside the source material collecting barrel unit; and a first airtight packing-plunger connector element that has one end portion to which the first airtight packing element is coupled and fixed and the other end portion to which the plunger element is coupled and fixed such that the first airtight packing element is moved forward and backward simultaneously along with forward and backward movement of the plunger element, wherein the target material extracting means coupling module is configured to include: a source material passage projecting element which extends and projects from the source material collecting space module by a certain length and is inserted into and fixed to the target material extracting means so as to be coupled to the target material extracting means; a step element which is provided to form a step by being positioned between the source material passage projecting element and the source material collecting space module and having a diameter larger than an outer diameter of the source material passage projecting element; and a source material flowing passage element which penetrates the source material passage projecting element and is connected to the source material collecting space module so as to enable a source material to flow into the source material collecting space module, and thereby the source material can be easily collected.

In addition, the target material extracting means is configured to include: a target material containing barrel unit in which a source material including a target material separated by the centrifugal separator is collected; and a target material discharging plunger unit which is inserted into the target material containing barrel unit to form the airtight target material containing barrel unit and is moved forward and backward depending on an operation of an operator to generate pressure inside the target material containing barrel unit, wherein the target material containing barrel unit is configured to include: a target material containing space module in which a source material including a target material separated by the centrifugal separator is stored; and a source material collecting means coupling module which is formed to project from an end portion of the target material containing space module such that an end portion of the source material collecting means is inserted into and coupled to the source material collecting means coupling module, wherein the target material discharging plunger unit is configured to include: a second airtight packing element which seals an inside of the target material containing barrel unit to form the airtight inside; and a second airtight packing-plunger connector element that has one end portion to which the second airtight packing element is coupled and fixed and the other end portion to which a plunger element is coupled and fixed such that the second airtight packing element is moved forward and backward simultaneously along with forward and backward movement of the plunger element, wherein the source material collecting means coupling module is configured to include: a target material passage projecting element that extends and projects from the target material containing space module by a certain length so as to be coupled to the source material collecting means by enabling the end portion of the source material collecting means to be inserted into and fixed in the target material passage projecting element; a target material flowing passage element that penetrates the target material passage projecting element and is connected to the target material containing space module so as to enable a target material to be extracted from a separated source material in the target material containing space module; and a leakage preventive space element that is formed at an end portion of the target material flowing passage element, into which the end portion of the source material collecting means is inserted, and that has a diameter larger than an outer diameter of the target material flowing passage element so as to inhibit a source material or a target material from leaking outside due to surface tension when the source material collecting means is decoupled, and thereby the target material can be easily extracted from the separated source material contained in the target material containing barrel unit.

In the meantime, according to another aspect of the present invention so as to accomplish these objects, there is provided to a method for extracting a target material by using a target material extraction kit for a centrifugal separator, the method including: a source material collecting step of collecting a source material of a subject by using a source material collecting means; and a target material extracting step of extracting the target material from the source material separated by the centrifugal separator by using a target material extracting means after the source material collecting step, wherein the source material collecting step is configured to include: a collecting means preparing step of preparing the source material collecting means so as to collect the source material from the subject; and a source material filling step of collecting the source material from the subject by using the source material collecting means, wherein the target material extracting step is configured to include: an extracting means preparing step of preparing the target material extracting means; an extracting means coupling step of coupling the target material extracting means to the source material collecting means filled with the source material collected from the subject; a centrifugation preparing step of moving the source material, with which the source material collecting means is filled, into the target material extracting means coupled to the source material collecting means by applying pressure to the source material collecting plunger unit; a plunger element decoupling step of decoupling a plunger element from the source material collecting barrel unit of the source material collecting means before the source material collecting means is inserted into the centrifugal separator; a centrifuging step of separating a source material through centrifugation by inserting the source material collecting means, from which the source material collecting plunger unit is decoupled, and the target material extracting means into the centrifugal separator; a plunger element coupling step of coupling and fixing the plunger element to a target material discharging plunger unit of the target material extracting means filled with the source material separated into layers by the centrifugal separator; a primary target material extracting step of injecting a part of the source material separated into layers, with which a target material containing barrel unit is filled, into the source material collecting barrel unit by applying pressure to the plunger element coupled and fixed to the target material discharging plunger unit to move the target material discharging plunger unit forward; a syringe body coupling step of decoupling the source material collecting means from the target material extracting means after the primary target material extracting step and coupling and fixing a syringe body which is to be filled with a target material, to the target material extracting means; a secondary target material extracting step of injecting a part of the source material separated into layers, which remains in the target material containing barrel unit, into the syringe body by re-applying pressure to the plunger element coupled and fixed to the target material discharging plunger unit to move the target material discharging plunger unit forward; and a target material preparation finishing step of decoupling the syringe body from the target material extracting means and finishing a target material extracting operation, thereby the target material can be easily extracted from the source material.

In the meantime, it should be understood that the terminology or the words used in claims should not be interpreted in normally or lexically sense. It should be interpreted as meaning and concept consistent with the technical idea of the present invention, based on the principle that the inventor can properly define the concept of the term in order to describe its invention in the best way.

Therefore, the embodiments described in the present specification and the configurations shown in the drawings are only the most preferred embodiments of the present invention, and not all the technical ideas of the present invention are described. Therefore, it is to be understood that various equivalents and modifications are possible.

### Advantageous Effects

According to the present invention, as described above, the configurations and operations provided above have the following effects.
1. A source material collecting means (100) and a target material extracting means (200) enable a target material to be extracted from a source material even through centrifugation performed only once.
2. In addition, the source material collecting means (100) and the target material extracting means (200) enable an extraction operation process using an additional needle for extracting a target material from a source material to be omitted.
3. In addition, the source material collecting means (100) and the target material extracting means (200) enable an additional dropping extraction operation process for extracting a target material from a source material to be omitted.
4. In addition, the source material collecting means (100) and the target material extracting means (200) enable an amount of target material extracted from a source material to be increased.
   In other words, the target material can be easily extracted from the source material even through centrifugation performed only once without using a needle.
5. In addition, the present invention is very effective in that since, during an extraction operation, the number of times of the centrifugation for separating the target material from the source material is reduced to once and a needling operation of extracting the target material by using a needle and a dropping operation of dropping a part of source material into an additional instrument little by little so as to extract the target material from the source material are omitted, an extraction time and an operation time are reduced, and an extraction operation environment is also cleanly and hygienically maintained by omitting the needling operation and the dropping operation.

### Brief Description of Drawings

The above and other objects, features and advantages of the present invention will be more apparent from the following detailed description taken in conjunction with the accompanying drawings, in which:
FIG. 1 is a configurational diagram illustrating a target material extraction kit for a centrifugal separator of the present invention;
FIG. 2 is a perspective view illustrating an embodiment (first embodiment) of the target material extraction kit for a centrifugal separator of the present invention;
FIG. 3 is a flowchart illustrating a method for extracting a target material by using the target material extraction kit for a centrifugal separator according to the present invention;
FIG. 4 is a conceptual diagram illustrating a flowchart for a method for extracting a target material by using the target material extraction kit for a centrifugal separator according to the present invention;
FIG. 5 is a perspective view illustrating further another embodiment (third embodiment) of the target material extraction kit for a centrifugal separator of the present invention; and

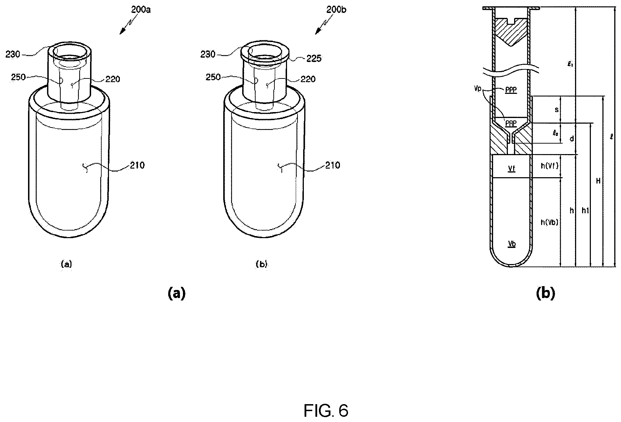

FIG. 6 illustrates a representative diagram of the prior art for a target material extraction kit for a centrifuge and a method for extracting a target material using the same according to the present invention.

### [Reference Signs List]

1: target material extraction kit for a centrifugal separator
2: method for extracting a target material by using the target material extraction kit for a centrifugal separator
100: source material collecting means
110: source material collecting barrel unit
111: source material collecting space module
111a: first slope surface
112: target material extracting means coupling module
112a: source material passage projecting element
112b: step element
112c: source material flowing passage element
113: first barrel handle
114: first airtight projection element
120: source material collecting plunger unit
121: first airtight packing element
122: plunger element
123: first airtight packing-plunger connector element
200: target material extracting means
210: target material containing barrel unit
211: target material containing space module
211a: second slope surface
212: source material collecting means coupling module
212a: target material passage projecting element
212b: target material flowing passage element
212c: leakage preventive space element
213: plunger fine adjustment zone module
214: second airtight projection element
220: target material discharging plunger unit
221: second airtight packing element
222: second airtight packing-plunger connector element
S100: source material collecting step
S110: collecting means preparing step
S120: source material filling step
S200: target material extracting step
S210: extracting means preparing step
S220: extracting means coupling step
S230: centrifugation preparing step
S240: plunger element decoupling step
S250: centrifuging step
S260: plunger element coupling step
S270: primary target material extracting step
S280: syringe body coupling step
S290: secondary target material extracting step
S300: target material preparation finishing step
SY: syringe body
a₁: angle of first slope surface (111a)
a₂: angle of second slope surface (211a)
122': target material extracting operation-dedicated plunger element

### Best Mode

### Mode for Invention

Hereinafter, functions, configurations, and operations of a target material extraction kit (1) for a centrifugal separator and a method (2) for extracting a target material by using the same of the present invention will be described in detail with reference to the accompanying drawings.

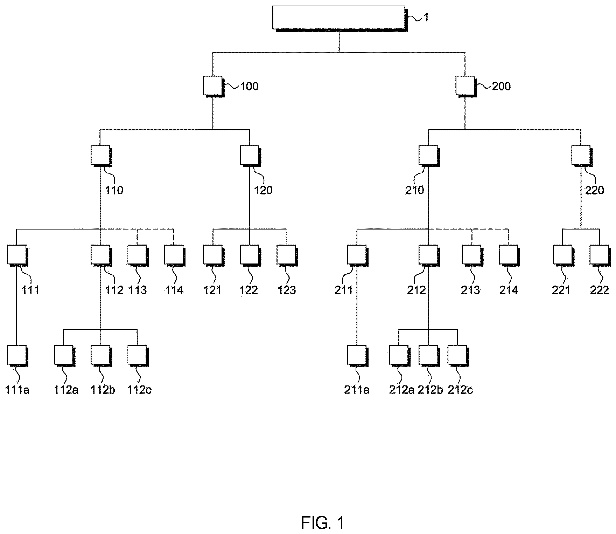

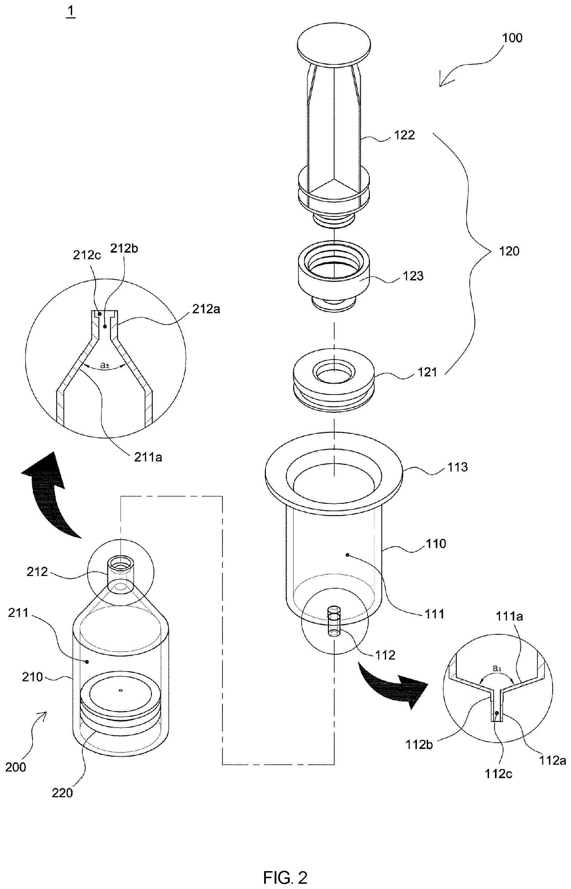

FIG. 1 is a configurational diagram illustrating a target material extraction kit for a centrifugal separator of the present invention, and FIG. 2 is a perspective view illustrating an embodiment (first embodiment) of the target material extraction kit for a centrifugal separator of the present invention.

According to the present invention, as illustrated in FIGS. 1 and 2, the target material extraction kit (1) for a centrifugal separator is configured to include: a source material collecting means (100) that collects a certain amount of source material; and a target material extracting means (200) that is coupled and fixed to an end portion of the source material collecting means (100) and is inserted into the centrifugal separator together with the source material collecting means (100).

The target material is to be easily separated and extracted from the source material separated into the layers formed inside the source material collecting means (100) and the target material extracting means (200) by the centrifugal separator.

In other words, the present invention relates to the target material extraction kit (1) for a centrifugal separator which enables a target material to be extracted from a source material even through centrifugation performed only once by using the source material collecting means (100) and the target material extracting means (200) and enables an extraction operation to be hygienically performed in terms of an extraction operation environment, thereby resulting in not only easiness of the extraction operation but also improvement in the extraction operation environment.

More specifically, a first embodiment is described as follows. The source material collecting means (100) that is a means for collecting a certain amount of source material including a target material from a subject is configured to include: a source material collecting barrel unit (110) which is to be filled with the certain amount of source material; and a source material collecting plunger unit (120) which is inserted into the source material collecting barrel unit (110), is moved forward and backward, and generates pressure by moving forward and backward to fill the source material collecting barrel unit (110) with the certain amount of source material.

The source material collecting barrel unit (110) is configured to include: a source material collecting space module (111) in which the certain amount of source material is stored; and a target material extracting means coupling module (112) which is formed to project from one end portion of the source material collecting space module (111) such that a part of the target material extracting means coupling module is inserted into and coupled to the target material extracting means (200).

The source material collecting plunger unit (120) is configured to include: a first airtight packing element (121) which seals an inside of the source material collecting barrel unit (110) to form the airtight inside; a plunger element (122) which moves the first airtight packing element (121) forward and backward inside the source material collecting barrel unit (110); and a first airtight packing-plunger connector element (123) that has one end portion to which the first airtight packing element (121) is coupled and fixed and the other end portion to which the plunger element (122) is coupled and fixed such that the first airtight packing element (121) is moved forward and backward simultaneously along with forward and backward movement of the plunger element (122).

The target material extracting means coupling module (112) is configured to include: a source material passage projecting element (112a) which extends and projects from the source material collecting space module (111) by a certain length and is inserted into and fixed to the target material extracting means (200) so as to be coupled to the target material extracting means (200); a step element (112b) which is provided to form a step by being positioned between the source material passage projecting element (112a) and the source material collecting space module (111) and having a diameter larger than an outer diameter of the source material passage projecting element (112a); and a source material flowing passage element (112c) which penetrates the source material passage projecting element (112a) and is connected to the source material collecting space module (111) so as to enable a source material to flow into the source material collecting space module (111).

Accordingly, the source material can be easily collected from a subject.

In this case, the target material extracting means (200) that is coupled and fixed to one side of the source material collecting means (100) filled with the source material collected from the subject and is inserted into the centrifugal separator is configured to include: a target material containing barrel unit (210) in which the source material including a target material separated by the centrifugal separator is collected; and a target material discharging plunger unit (220) which is inserted into the target material containing barrel unit (210) to form the airtight target material containing barrel unit and is moved forward and backward depending on an operation of an operator to generate pressure inside the target material containing barrel unit (210).

The target material containing barrel unit (210) is configured to include: a target material containing space module (211) in which the source material including the target material separated by the centrifugal separator is stored; and a source material collecting means coupling module (212) which is formed to project from an end portion of the target material containing space module (211) such that an end portion of the source material collecting means (100) is inserted into and coupled to the source material collecting means coupling module.

The target material discharging plunger unit (220) is configured to include: a second airtight packing element (221) which seals an inside of the target material containing barrel unit (210) to form the airtight inside; and a second airtight packing-plunger connector element (222) that has one end portion to which the second airtight packing element (221) is coupled and fixed and the other end portion to which a plunger element (122) is coupled and fixed such that the second airtight packing element (221) is moved forward and backward simultaneously along with forward and backward movement of the plunger element (122).

The source material collecting means coupling module (212) is configured to include: a target material passage projecting element (212a) that extends and projects from the target material containing space module (211) by a certain length so as to be coupled to the source material collecting means (100) by enabling the end portion of the source material collecting means (100) to be inserted into and fixed in the target material passage projecting element; a target material flowing passage element (212b) that penetrates the target material passage projecting element (212a) and is connected to the target material containing space module (211) so as to enable the target material to be extracted from the separated source material in the target material containing space module (211); and a leakage preventive space element (212c) that is formed at an end portion of the target material flowing passage element (212b), into which the end portion of the source material collecting means (100) is inserted, and that has a diameter larger than an outer diameter of the target material flowing passage element (212b) so as to inhibit the source material or the target material from leaking outside due to surface tension when the source material collecting means (100) is decoupled.

Accordingly, the target material can be easily extracted from the separated source material contained in the target material containing barrel unit (210).

In other words, the source material is collected from the subject by using the source material collecting means (100) described above, and the target material extracting means coupling module (112) is inserted into and fixed to the source material collecting means coupling module (212) of the target material extracting means (200) in a state where the source material collecting space module (111) is filled with the collected source material, such that the source material, which is filled in the source material collecting space module (111), is separated by the centrifugal separator and is to be located in both the source material collecting space module (111) and the target material containing space module (211).

As described above, the source material collecting means (100) and the target material extracting means (200) which are coupled to each other by an operator is inserted into the centrifugal separator with the target material extracting means (200) being positioned at a bottom side, and then the source material is subjected to centrifugation.

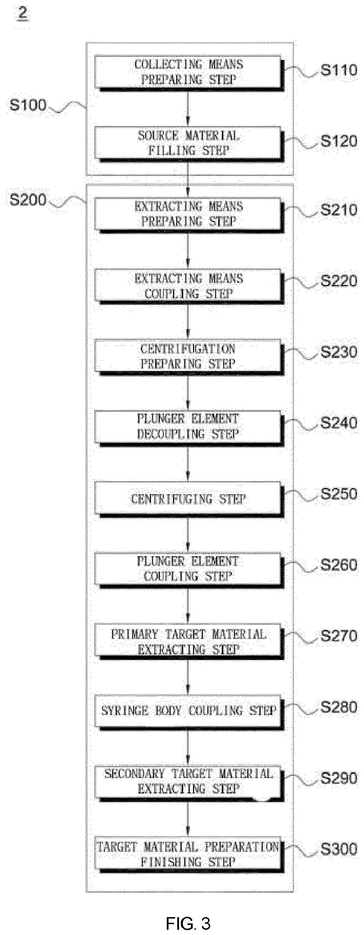

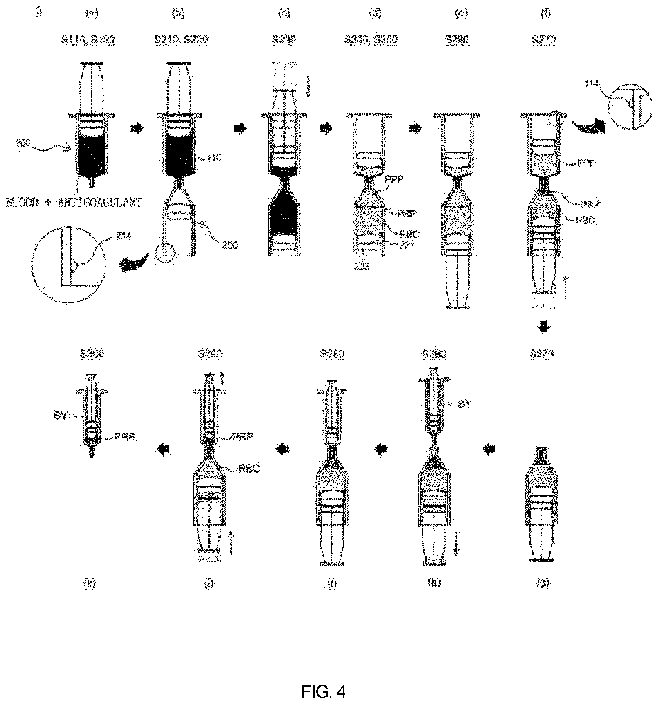

In this manner, the source material is separated into layers formed in the source material collecting space module (111) and the target material containing space module (211) by the centrifugal separator, and the target material is to be extracted from the source material having the layers, as illustrated by an embodiment in FIGS. 3 and 4.

Hereinafter, a configuration of the target material extraction kit (1) for a centrifugal separator of the present invention will more specifically described. The plunger element (122) that is coupled to and decoupled from the first airtight packing-plunger connector element (123) and the second airtight packing-plunger connector element (222) according to a procedure of an operation (2) for extracting a target material can be formed by a male and female thread coupling method such that an operator can easily couple and decouple the plunger element to and from the first airtight packing-plunger connector element (123) and the second airtight packing-plunger connector element (222).

In addition, as illustrated in FIG. 2, the source material collecting barrel unit (110) of the source material collecting means (100) and the target material containing barrel unit (210) of the target material extracting means (200) have respective spaces having a certain volume so as to be filled with a source material and a target material and are opened and airtightly sealed by the source material collecting plunger unit (120) and the target material discharging plunger unit (220), respectively.

In addition, the source material collecting barrel unit (110) is configured to further include: a first barrel handle (113) which is formed to project in a horizontal direction by a certain length from an outer circumferential edge of the other end portion of the source material collecting space module (111) such that an operator easily and stably handles the source material collecting barrel unit (110) and the source material collecting plunger unit (120); and a first airtight projection element (114) which is formed to project in a horizontal direction by a certain length from one side of an inner circumferential edge of the other end portion of the source material collecting space module (111) so as not only to maximize airtightness and hermeticity obtained by the source material collecting plunger unit (120) but also to inhibit the source material collecting plunger unit (120) from being freely decoupled from the source material collecting space module (111).

Accordingly, the operator can easily handle the source material collecting means (100).

The target material containing barrel unit (210) is also configured to further include, as the same function as that of the first airtight projection element, a second airtight projection element (214) which is formed to project in the horizontal direction by a certain length from one side of an inner circumferential edge of the other end portion of the target material containing space module (211) so as not only to maximize airtightness and hermeticity obtained by the target material discharging plunger unit (220) but also to inhibit the target material discharging plunger unit (220) from being freely decoupled from the target material containing space module (211).

Accordingly, the operator can easily handle the target material extracting means (200).

In this case, the free decoupling of the source material collecting plunger unit (120) from the source material collecting space module (111) and the target material discharging plunger unit (220) from the target material containing space module (211) means easy decoupling even by a relatively weak force and means the decoupling by an unintentional force other than a force which is applied by an operator to intentionally decouple the source material collecting plunger unit (120) and the target material discharging plunger unit (220) from the source material collecting space module (111) and the target material containing space module (211), respectively.

In addition, the source material collecting space module (111) has a first slope surface (111a) on one end portion thereof such that movement of the source material collecting plunger unit (120), movement of a source material, and movement of a target material are easily performed.

In other words, if the one end portion of the source material collecting space module (111) connected to the source material flowing passage element (112c) is formed in a flat surface, the source material collecting plunger unit (120) moves only when high pressure is momentarily generated. Hence, an operator cannot completely control the movement of the source material collecting plunger unit (120).

Consequently, the pressure inside the source material collecting space module (111) is distributed over the first slope surface (111a) such that the operator can completely control the movement of the source material collecting plunger unit (120).

Similarly, the target material containing space module (211) has a second slope surface (211a) on one end portion thereof such that movement of the target material discharging plunger unit (220), movement of a source material, and movement of a target material are easily performed.

In this case, the second slope surface (211a) is to be formed at an angle steeper than the first slope surface (111a) (a₁ ≥ a₂) so as to completely extract a small amount of target material.

In other words, a small amount of target material located in the target material containing space module (211) gradually rises as an operator applies pressure to the target material discharging plunger unit (220) such that a thickness of a layer of target material to be extracted by the operator is gradually increased as the target material is moved toward the end portion of the target material containing space module (211) which has a cross-sectional area that is gradually decreased due to the second slope surface (211a) (effect of maximizing visibility of the target material).

Consequently, the second slope surface (211a) distributes the pressure inside the target material containing space module (211), similarly to the function of the first slope surface (111a), such that not only the operator can completely control the movement of the target material discharging plunger unit (220), but also the visibility of the target material to be extracted is maximized.

The above-described process is to be more specifically described with a method (2) for extracting a target material by using the target material extraction kit for a centrifugal separator.

As simply illustrated in FIG. 4, the method (2) for extracting a target material by using the target material extraction kit for a centrifugal separator is configured to include: a source material collecting step (S100) of collecting a source material of a subject by using the source material collecting means (100); and a target material extracting step (S200) of extracting the target material from the source material separated by the centrifugal separator by using the target material extracting means (200) after the source material collecting step (S100). The source material collecting step (S100) is configured to include: a collecting means preparing step (S110) of preparing the source material collecting means (100) so as to collect the source material from the subject; and a source material filling step (S120) of collecting the source material from the subject by using the source material collecting means (100).

The target material extracting step (S200) is configured to include: an extracting means preparing step (S210) of preparing the target material extracting means (200); an extracting means coupling step (S220) of coupling the target material extracting means (200) to the source material collecting means (100) filled with the source material collected from the subject; a centrifugation preparing step (S230) of moving the source material, with which the source material collecting means (100) is filled, into the target material extracting means (200) coupled to the source material collecting means (100) by applying pressure to the source material collecting plunger unit (120); a plunger element decoupling step (S240) of decoupling the plunger element (122) from the source material collecting barrel unit (110) of the source material collecting means (100) before the source material collecting means is inserted into the centrifugal separator; a centrifuging step (S250) of separating a source material through centrifugation by inserting the source material collecting means (100), from which the source material collecting plunger unit (120) is decoupled, and the target material extracting means (200) into the centrifugal separator; a plunger element coupling step (S260) of coupling and fixing the plunger element (122) to the target material discharging plunger unit (220) of the target material extracting means (200) filled with the source material separated into layers by the centrifugal separator; a primary target material extracting step (S270) of injecting a part of the source material separated into layers, with which the target material containing barrel unit (210) is filled, into the source material collecting barrel unit (110) by applying pressure to the plunger element (122) coupled and fixed to the target material discharging plunger unit (220) to move the target material discharging plunger unit forward; a syringe body coupling step (S280) of decoupling the source material collecting means (100) from the target material extracting means (200) after the primary target material extracting step (S270) and coupling and fixing a syringe body (SY), which is to be filled with a target material, to the target material extracting means (200); a secondary target material extracting step (S290) of injecting a part of the source material separated into layers, which remains in the target material containing barrel unit (210), into the syringe body (SY) by re-applying pressure to the plunger element (122) coupled and fixed to the target material discharging plunger unit (220) to move the target material discharging plunger unit forward; and a target material preparation finishing step (S300) of decoupling the syringe body (SY) from the target material extracting means (200) and finishing a target material extracting operation.

Accordingly, the target material can be easily extracted from the source material.

For example, when the source material is blood, and the target material which is to be extracted from the blood is platelet rich plasma (PRP) and platelet poor plasma (PPP), the extraction is as follows, with reference to FIG. 4.
(a) illustrates a state where a subject's blood is collected in the source material collecting means (100).
(b) illustrates a state where the source material collecting means (100), in which the subject's blood is collected, is coupled to the target material extracting means (200).
(c) illustrates a state where centrifugation is prepared by moving a certain amount of blood, with which the source material collecting means (100) is filled, into the target material extracting means (200) (a state where a certain amount of blood is moved into the target material extracting means (200) before the centrifugation).
(d) illustrates a state after the plunger element (122) is decoupled from the source material collecting barrel unit (110) from the state illustrated in (c) described above, the source material collecting means is inserted into the centrifugal separator, and the blood is subjected to the centrifugation.
(e) illustrates a state where the plunger element (122) decoupled from the source material collecting barrel unit (110) from (c) described above is coupled to the second airtight packing-plunger connector element (222), and a start of an extraction operation for extracting the target material is prepared.
(f) illustrates a state where the plunger element (122) in (e) is pressed in an arrow direction, and the platelet poor plasma (PPP) which is a first target material is extracted by an extraction operation performed by an operator.
(g) illustrates a state where the source material collecting means (100) filled with the platelet poor plasma (PPP) which is the first target material extracted from (f) is decoupled from the target material extracting means (200).
(h) illustrates a state of preparing a syringe body (SY) into which the platelet rich plasma (PRP) that is a secondary target material is to be extracted.
(i) illustrates a state where the syringe body (SY) is coupled to the target material extracting means (200).
(j) illustrates a state where the plunger element (122) coupled to the second airtight packing-plunger connector element (222) is re-pressed in the arrow direction, similarly to (f) described above, and the platelet rich plasma (PRP) which is a secondary target material is extracted into the syringe body (SY) by the extraction operation performed by an operator.
(k) illustrates a state where the syringe body (SY) filled with platelet rich plasma (PRP) which is the secondary target material is decoupled from the target material extracting means (200).

Then, the first target material (PPP) and the secondary target material (PRP) are both extracted from the blood, and remaining red blood cells remains as it is in the target material extracting means (200) to be easily disposed.

In this manner, in the method (2) for extracting a target material described above, a needling operation for extracting a target material by using a needle and a dropping operation for dropping a certain small amount of source material to the floor and removing the certain small amount of source material are omitted such that an extraction operation environment is cleanly and hygienically maintained, and easiness of the extraction operation of the target material is maximized.

For reference, in the operation (2) for extracting a target material by using the target material extraction kit for a centrifugal separator of the present invention, preparation of the target material extracting means (200) in the extracting means preparing step (S210) means that the target material discharging plunger unit (220) is prepared to be coupled to the source material collecting means (100) in a state where the target material discharging plunger unit is positioned at a specific position in the target material containing barrel unit (210) (the specific position of the target material discharging plunger unit (220) means that the target material discharging plunger unit (220) is positioned at the uppermost end in an opposite direction of a direction in which the target material extracting means (200) coupled to the source material collecting means (100) is inserted into the centrifugal separator).

The above-described process is performed such that minimum air remains inside the target material extracting means (200) which is to be coupled to the source material collecting means (100).

In other words, when pressure which is to be applied by the centrifugal separator to the inside of the source material collecting means (100) and the target material extracting means (200) is minimized, and the locations of the first airtight packing element (121) and the second airtight packing element (221) can be completely controlled when locations of the first airtight packing element (121) and the second airtight packing element (221) are simultaneously changed by the plunger element (122).

In addition, when the subject's blood is collected into the source material collecting means (100), a certain amount of anticoagulant has to be included (to inhibit coagulation of blood).

In addition, the 'source material' in the present invention can be mainly defined as blood, as described with the example in the above description of FIG. 4, and the 'target material' can be defined as the platelet rich plasma (PRP) and the platelet poor plasma (PPP) which can be obtained from blood.

As described above, the present invention is a result of modification of Prior Technology 1 that is applied and registered by the present applicants, and lack of description for a part other than the technical features of the present invention which are described in this specification is replaced with technical contents of Prior Technology 1.

In addition, compared to Prior Technology 1, the target material extraction kit (1) for a centrifugal separator of the present invention is configured as follows. A volume of the source material which can be collected into the source material collecting barrel unit (110) is 20 ml. A volume of the target material containing barrel unit (210) is increased to 12.5 ml. The target material containing barrel unit (210) contains 100% of red blood cells (RBC), 100% of platelet rich plasma (PRP), and a part of platelet poor plasma (PPP), and thereby the target material can be easily extracted from the source material by using the method (2) for extracting a target material as described above.

On the other hand, a second embodiment of the present invention has the same configuration as that of the first embodiment; however, the source material collecting means coupling module (212) formed at the target material containing barrel unit (210) has a structure of a luer-lock tip such that a coupling force to the target material extracting means coupling module (112) formed at the source material collecting barrel unit (110) can be improved and the sealing force is maximized so as to inhibit the source material and the target material from leaking.

In this case, when the source material collecting means coupling module (212) is configured to have a luer-lock tip structure, the target material extracting means coupling module (112) is also formed to have a structure corresponding thereto.

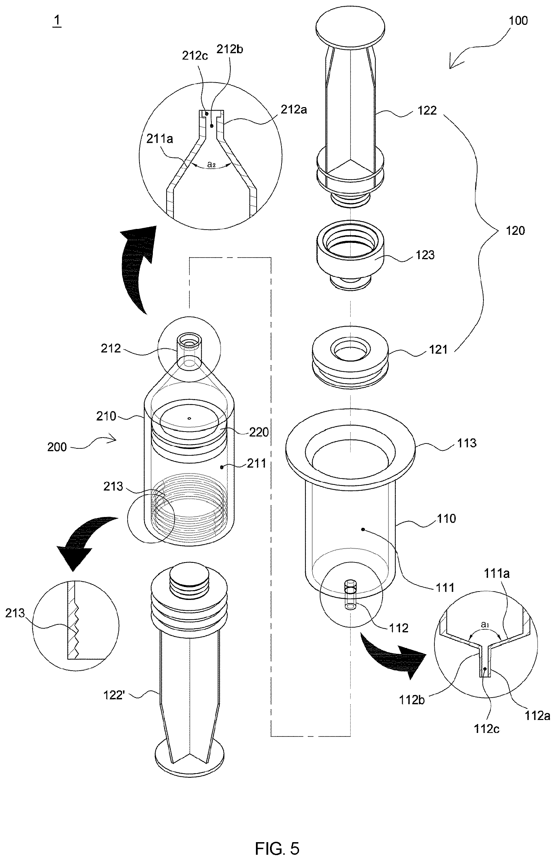

In addition, as another embodiment of the present invention, a third embodiment is configured to have the same configuration as that of any one embodiment of the first embodiment and the second embodiment described above. However, as illustrated in FIG. 5, the third embodiment is configured to further include: a target material extracting operation-dedicated plunger element (122') which is formed to have the same structure as that of the plunger element (122) which is inserted into the source material collecting barrel unit (110) and has a lower end portion which is formed to have a male thread shape in a certain zone such that insertion of the target material containing barrel unit (210) and the extraction operation of the target material are more accurately performed; and a plunger fine adjustment zone module (213) which is formed to have a female thread shape in a certain zone of an upper portion of an inner circumferential surface of the target material containing barrel unit (210) so as to correspond to the target material extracting operation-dedicated plunger element (122') such that forward and backward movement of the target material extracting operation-dedicated plunger element (122') is more accurately performed. Accordingly, accuracy, fineness, preciseness, and easiness of the extraction operation of the target material can be maximized.

As described above, the present invention is not limited to the described embodiment, and it is obvious for those who have common knowledge in the art to variously modify and change the present invention without departing from the idea and the scope of the present invention.

Hence, since the present invention can be realized as various embodiments without departing from the technical idea or the major feature, the embodiments of the present invention are only provided as simple examples and are not to be construed narrowly but can be variously modified.

### Industrial Applicability

The present invention relates to a target material extraction kit for a centrifugal separator and a method for extracting a target material by using the same and can be applied to a manufacturing business of manufacturing extraction kits and a sales business thereof, hospitals and research institutes where the operation for extracting the target material using the extraction kit according to the present invention is performed, and a promotion of the medical industry and the research industry.

## Claims

1. A target material extraction kit (1) for a centrifugal separator, comprising:
a source material collecting means (100) that collects a certain amount of source material; and
a target material extracting means (200) that is coupled and fixed to an end portion of the source material collecting means (100) and is inserted into the centrifugal separator together with the source material collecting means (100),
wherein the target material is to be easily separated and extracted from the source material separated into the layers formed inside the source material collecting means (100) and the target material extracting means (200) by the centrifugal separator.

2. The target material extraction kit for a centrifugal separator according to claim 1, wherein the source material collecting means (100) is configured to include:
a source material collecting barrel unit (110) which is to be filled with the certain amount of source material; and
a source material collecting plunger unit (120) which is inserted into the source material collecting barrel unit (110), moves forward and backward, and generates pressure by moving forward and backward to cause the source material collecting barrel unit (110) to be filled with the certain amount of source material,
wherein the source material collecting barrel unit (110) is configured to include:
a source material collecting space module (111) in which the certain amount of source material is stored; and
a target material extracting means coupling module (112) which is formed to project from an end portion of the source material collecting space module (111) such that a part of the target material extracting means coupling module is inserted into and coupled to the target material extracting means (200),
wherein the source material collecting plunger unit (120) is configured to include:
a first airtight packing element (121) which seals an inside of the source material collecting barrel unit (110) to form the airtight inside;
a plunger element (122) which moves the first airtight packing element (121) forward and backward inside the source material collecting barrel unit (110); and
a first airtight packing-plunger connector element (123) that has one end portion to which the first airtight packing element (121) is coupled and fixed and the other end portion to which the plunger element (122) is coupled and fixed such that the first airtight packing element (121) is moved forward and backward simultaneously along with forward and backward movement of the plunger element (122),
wherein the target material extracting means coupling module (112) is configured to include:
a source material passage projecting element (112a) which extends and projects from the source material collecting space module (111) by a certain length and is inserted into and fixed to the target material extracting means (200) so as to be coupled to the target material extracting means (200);
a step element (112b) which is provided to form a step by being positioned between the source material passage projecting element (112a) and the source material collecting space module (111) and having a diameter larger than an outer diameter of the source material passage projecting element (112a); and
a source material flowing passage element (112c) which penetrates the source material passage projecting element (112a) and is connected to the source material collecting space module (111) so as to enable a source material to flow into the source material collecting space module (111), and
thereby the source material can be easily collected.

3. The target material extraction kit for a centrifugal separator according to claim 1, wherein the target material extracting means (200) is configured to include:
a target material containing barrel unit (210) in which a source material including a target material separated by the centrifugal separator is collected; and
a target material discharging plunger unit (220) which is inserted into the target material containing barrel unit (210) to form the airtight target material containing barrel unit and is moved forward and backward depending on an operation of an operator to generate pressure inside the target material containing barrel unit (210),
wherein the target material containing barrel unit (210) is configured to include:
a target material containing space module (211) in which a source material including a target material separated by the centrifugal separator is stored; and
a source material collecting means coupling module (212) which is formed to project from an end portion of the target material containing space module (211) such that an end portion of the source material collecting means (100) is inserted into and coupled to the source material collecting means coupling module,
wherein the target material discharging plunger unit (220) is configured to include:
a second airtight packing element (221) which seals an inside of the target material containing barrel unit (210) to form the airtight inside; and
a second airtight packing-plunger connector element (222) that has one end portion to which the second airtight packing element (221) is coupled and fixed and the other end portion to which a plunger element (122) is coupled and fixed such that the second airtight packing element (221) is moved forward and backward simultaneously along with forward and backward movement of the plunger element (122),
wherein the source material collecting means coupling module (212) is configured to include:
a target material passage projecting element (212a) that extends and projects from the target material containing space module (211) by a certain length so as to be coupled to the source material collecting means (100) by enabling the end portion of the source material collecting means (100) to be inserted into and fixed in the target material passage projecting element;
a target material flowing passage element (212b) that penetrates the target material passage projecting element (212a) and is connected to the target material containing space module (211) so as to enable a target material to be extracted from a separated source material in the target material containing space module (211); and
a leakage preventive space element (212c) that is formed at an end portion of the target material flowing passage element (212b), into which the end portion of the source material collecting means (100) is inserted, and that has a diameter larger than an outer diameter of the target material flowing passage element (212b) so as to inhibit a source material or a target material from leaking outside due to surface tension when the source material collecting means (100) is decoupled, and
thereby the target material can be easily extracted from the separated source material contained in the target material containing barrel unit (210).

4. A method (2) for extracting a target material by using a target material extraction kit for a centrifugal separator, the method comprising:
a source material collecting step (S100) of collecting a source material of a subject by using a source material collecting means (100); and
a target material extracting step (S200) of extracting the target material from the source material separated by the centrifugal separator by using a target material extracting means (200) after the source material collecting step (S100),
wherein the source material collecting step (S100) is configured to include:
a collecting means preparing step (S110) of preparing the source material collecting means (100) so as to collect the source material from the subject; and
a source material filling step (S120) of collecting the source material from the subject by using the source material collecting means (100),
wherein the target material extracting step (S200) is configured to include:
an extracting means preparing step (S210) of preparing the target material extracting means (200);
an extracting means coupling step (S220) of coupling the target material extracting means (200) to the source material collecting means (100) filled with the source material collected from the subject;
a centrifugation preparing step (S230) of moving the source material, with which the source material collecting means (100) is filled, into the target material extracting means (200) coupled to the source material collecting means (100) by applying pressure to the source material collecting plunger unit (120);
a plunger element decoupling step (S240) of decoupling a plunger element (122) from the source material collecting barrel unit (110) of the source material collecting means (100) before the source material collecting means is inserted into the centrifugal separator;
a centrifuging step (S250) of separating a source material through centrifugation by inserting the source material collecting means (100), from which the source material collecting plunger unit (120) is decoupled, and the target material extracting means (200) into the centrifugal separator;
a plunger element coupling step (S260) of coupling and fixing the plunger element (122) to a target material discharging plunger unit (220) of the target material extracting means (200) filled with the source material separated into layers by the centrifugal separator;
a primary target material extracting step (S270) of injecting a part of the source material separated into layers, with which a target material containing barrel unit (210) is filled, into the source material collecting barrel unit (110) by applying pressure to the plunger element (122) coupled and fixed to the target material discharging plunger unit (220) to move the target material discharging plunger unit forward;
a syringe body coupling step (S280) of decoupling the source material collecting means (100) from the target material extracting means (200) after the primary target material extracting step (S270) and coupling and fixing a syringe body (SY) which is to be filled with a target material, to the target material extracting means (200);
a secondary target material extracting step (S290) of injecting a part of the source material separated into layers, which remains in the target material containing barrel unit (210), into the syringe body (SY) by re-applying pressure to the plunger element (122) coupled and fixed to the target material discharging plunger unit (220) to move the target material discharging plunger unit forward; and
a target material preparation finishing step (S300) of decoupling the syringe body (SY) from the target material extracting means (200) and finishing a target material extracting operation,
thereby the target material can be easily extracted from the source material.
